# EUROPEAN PATENT APPLICATION

(11) **EP 4 656 178 A1**
(43) Date of publication of application: **03.12.2025**
(21) Application number: 24382565.0
(22) Date of filing: 27.05.2024
(51) Int. Cl.: A61K 8/97, A61K 8/9789, A61Q 19/08

(54) **COSMETIC USE OF ATRACTYLODES LANCEA ROOT EXTRACT**

(71) Applicant: Provital, S.A., 08210 Barberà del Vallès (ES)
(72) Inventor: MORENO RAJA, Miguel Ramón, 08025 BARCELONA (ES); ASO PÉREZ, Miguel, 08191 RUBÍ (ES); MANZANO ALÍAS, David, 08025 BARCELONA (ES)
(74) Representative: Sugrañes, S.L.P.

(57) **Abstract**

It refers to the cosmetic use of *Atractylodes lancea* root extract for skin anti-aging. It refers also to a cosmetic method for skin anti-aging. The extract is suitable for skin anti-aging showing a retinol-like functionality, but without exhibiting the disadvantages thereof.

## Description

### FIELD OF THE INVENTION

The present invention relates to the field of cosmetic compositions for skin anti-aging.

### BACKGROUND ART

Skin aging is the progressive biological process generally characterized by the appearance of wrinkles, age spots, sagging of skin, and dryness.

Skin aging is a complex biological process influenced by a combination of endogenous (genetics, cellular metabolism, hormone and metabolic processes) and exogenous (chronic light exposure, pollution, ionizing radiation, chemicals, toxins) factors. These factors lead together to cumulative structural and physiological alterations and progressive changes in each skin layer as well as changes in skin appearance, especially, on the sun-exposed skin areas.

Since skin is an essential part of physical appearance, this has led to increased concerns about skin care. Anti-aging products help improve the quality and health of the skin by nourishing it.

Different previous approaches have been reported to improve skin health and counteract the effects of aging.

In US-A-2009/0036545 it is disclosed the use of bakuchiol, a known plant-based retinol alternative, which is obtained from *Psoralea coryfolia,* and which is used in a method for treating, preventing and improving the condition and/or aesthetic appearance of aging skin, particularly, treating, preventing, ameliorating, reducing and/or eliminating fine lines and/or wrinkles of skin, through meroterpene induced gene manipulation.

In Mane et al., Skin anti-ageing strategies: a review, Int. J. Eng. App., Sci, Technol., 2019, 4(7), 255-263, it is disclosed that the skin anti-aging strategies attempted to reverse the dermal and epidermal signs of photo- and chronological aging can be grouped under the following approaches: cosmetological care and topical agents. The former includes daily skin care, correct sun protection and aesthetic non-invasive procedures, and the latter includes the use of antioxidants (e.g., vitamins, polyphenols and flavonoids), which reduce collagen degradation, and cell regulators (e.g., retinols, peptides, and growth factors), which have direct effects on collagen metabolism and influence collagen production.

In Brown et al., Natural Retinol Analogs Potentiate the Effects of Retinal on Aged and Photodamaged Skin: Results from In Vitro to Clinical Studies, Dermatol. Ther. (Heidelb), 2023, 13, 2299-2317, it is disclosed that the combination of bakuchiol, *Vigna aconitifolia* extract and retinal is a highly effective and well-tolerated treatment for photoaging.

In T. Quan, Human skin aging and the anti-aging properties of retinol, Biomolecules, 2023, 13, 1614, it is disclosed that retinol, a derivative of vitamin A, has demonstrated its effectiveness in promoting various anti-aging benefits for the skin and is predominantly favoured within the category of retinoids, because it can stimulate collagen synthesis, reduce oxidative stress, and modulate gene expression. It is also disclosed that inappropriate or excessive use of topical retinol may also result in potential side effects, and that the consumption of excessive amounts of oral retinol derivatives used can lead to detrimental consequences, including symptoms like nausea, vomiting, headaches, and dizziness.

Although retinol has dominated since the 1970s as anti-aging active, its irritation potential has triggered the need for new actives more efficient and without showing the disadvantages of retinol.

### SUMMARY OF INVENTION

An aspect of the present invention relates to the cosmetic use of an *Atractylodes lancea* root extract (ALRE) for skin anti-aging, wherein said extract is the only anti-aging active.

Another aspect of the invention is a cosmetic method for skin anti-aging.

### FIGURES

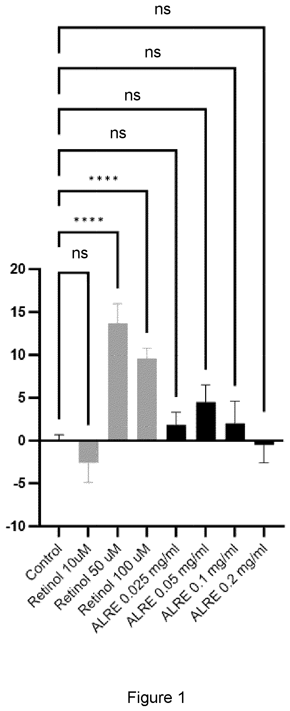

Figure 1 shows the activity of the human TRPV1 channel following a 1-hour incubation with varying concentrations of either retinol or the ALRE. Bar charts represent the percentage of intracellular calcium increase of retinol and the ALRE, after 1-hour incubation. Data is normalized to the mean maximal fluorescence signal induced by a direct application of 1µM capsaicin on cycle 10 (considered as 100%) and fluorescence signal change produced by assay buffer (control as 0%). Data is represented as mean ± SEM. Statistical analysis consisted in one-way ANOVA with unmatched measures followed by Dunnett's multiple comparisons post hoc test. Statistical significance was set to p<0.05; (*)p<0.05, (***)p<0.001, (****)p<0.0001. Number of technical replicates (n)=3, number of independent experiments (N) =3.
Figure 2 shows the modulation of gene expression related to "Retinoid binding, metabolizing genes and lipid transport" by retinol and the ALRE. Relative gene expression data is expressed as % change over non-treated human fibroblast. Control expression is considered to be 100%.
Figure 3 shows the modulation of gene expression related to Collagens by Retinol (RET) and the ALRE. Relative gene expression data is expressed as % change over non-treated human fibroblast. Control expression is considered to be 100%.
Figure 4 shows the modulation of gene expression related to "Extracellular matrix (ECM)" by Retinol (RET) and the ALRE. Relative gene expression data is expressed as % change over non-treated human fibroblast. Control expression is considered to be 100%.
Figure 5 shows the modulation of gene expression related to "Matrix Metalloproteases (MMPs) and Pro-inflammatory genes" by Retinol (RET) and the ALRE. Relative gene expression data is expressed as % change over non-treated human fibroblast. Control expression is considered to be 100%.

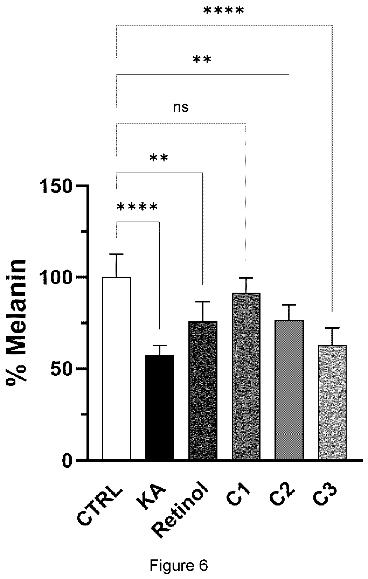

Figure 6 shows the percentage of melanin reduction, in comparison with Control, in reconstructed human pigmented epidermis (RHPE) after daily application of the ALRE from day 9 to day 17. The melanin quantification was performed by imaging. Control (CTRL) represents the RHPE model untreated, KA: treated with 250 µM kojic acid; Retinol: with 10 µM retinol and C1, C2 and Ce treated with different concentration of the ALRE (C1= 0.05 mg/ml; C2 = 0.1 mg/ml y C3 = 0.2 mg/ml). Statistical analysis consisted in one-way analysis of variance (ANOVA) with unmatched measures followed by Tukey's multiple comparisons test. Statistical significance was set to ** p<0.01; *** p<0.001 and **** p<0.0001. Number of independent experiments N = 3.

### DESCRIPTION OF THE INVENTION

The present invention relates to the cosmetic use of an *Atractylodes lancea* root extract (ALRE) for skin anti-aging by topical application, wherein said extract is the only anti-aging active.

The inventors of the present invention have developed an *Atractylodes lancea* root extract, whose cosmetic use is suitable for skin anti-aging, which surprisingly shows improved features in comparison to retinol, one of the most used anti-aging active.

In the present description, as well as in the claims, the singular forms "a", "an" and "the" include the plural reference unless the context clearly indicates otherwise. The term "about" refers to a deviation of plus/minus 10%, preferably plus/minus 5%. The percentages are expressed in % by weight (wt.%), unless stated the contrary. The ranges defined by the terms "between ... and ..." or by the terms "from ...to..." are meant to include also said stated endpoints thereof, and they also include any narrower sub-range.

### Use of the ALRE

The object of the present invention is the cosmetic use of an *Atractylodes lancea* root extract (ALRE) for skin anti-aging by topical application, wherein said extract is the only anti-aging active.

As used herein, "cosmetic use" is defined as a product intended to be applied to the human skin for cleansing, beautifying, promoting attractiveness, or altering the appearance without affecting the body's structure or functions. In an embodiment, it is defined as a product intended to improve and/or embellish any part of the body showing a deficit in collagen or other components of the extracellular matrix (ECM), such as, for example, wrinkles on face or hands, neckline, lips, cheeks, or eyelids.

As used herein, "topical application" means directly laying on or spreading on outer skin using, e.g., the hands or an applicator such as a wipe, puff, roller, spray, or cosmetotextiles. Cosmetotextiles are textiles, which provide cosmetic and biological functions, such as pleasant feeling, energising, slimming, refreshing, vitalising, skin glowing, anti-ageing, body care, fitness and health.

In the scope of this description, the term "skin anti-aging" refers to the ability of certain actives/treatments to counteract the effects of aging on the skin and promote a younger, healthier appearance. The capacity of these assets/treatments has to do with the following activities:
- As shown in Example 1, the use of the ALRE according to the present invention does not activate and sensitize the nociceptors, as done by retinoids, which are susceptible to induce pain and neurogenic inflammation.
- As shown in Example 2.1, many of the significantly upregulated genes exhibited similar levels of expression by the presence of retinol or the ALRE.
- As shown in Example 2.2, the gene upregulation by the ALRE significantly surpasses retinol in its impact on these seven types of collagen-related genes, indicating a superior ability to enhance the skin's extracellular matrix.
- As shown in Example 2.3, the action of the ALRE is poised to support the preservation of youthful skin characteristics by attenuating the collagen-degrading effects of matrix metalloproteinase enzymes.

Surprisingly, the use of the ALRE according to the present invention shows an improved performance over retinol regarding different factors affecting the skin health, making suitable its use for anti-aging treatments. The ALRE is surprisingly suitable for skin anti-aging showing a retinol-like functionality, but without exhibiting the disadvantages of retinol. One advantage is that the ALRE does not induce pain and neurogenic inflammation, as indicated by the absence of activation of transient receptor potential vanilloid 1 (TRPV1). Thus, surprisingly, the ALRE is a plant-based retinol alternative for anti-aging treatments.

In an embodiment, the use of the ALRE is selected from for stimulating collagen synthesis, for not inducing MMP activity, for smoothing wrinkles, for reducing the oxidative stress, and for modulating of gene expression to influence the expression of genes related to the health and proper functioning of the skin, such as, retinoid binding, metabolizing genes, lipid transport, and extracellular matrix.

In an embodiment, the use of the ALRE is for stimulating collagen synthesis. Collagen is a key protein in the skin that provides structure and firmness. The increase of collagen production helps improving the skin's elasticity and firmness.

In an embodiment, the use of the ALRE is for not inducing MMP activity. Matrix metalloproteinases (MMPs) are enzymes that can degrade collagen and other components of the skin's extracellular matrix, leading to the formation of wrinkles and loss of elasticity. Preventing the gene expression or inhibition of the activity of these enzymes helps protecting the structure of the skin.

In an embodiment, the use of the ALRE is for smoothing wrinkles.

In an embodiment, the use of the ALRE is for reducing the oxidative stress. Oxidative stress, caused by the buildup of free radicals in the skin, contributes to premature aging by damaging skin cells and structures. An antioxidant activity helps reducing this oxidative stress, thus protecting the skin from damage.

In an embodiment, the use of the ALRE is for modulating of gene expression to influence the expression of genes related to the health and proper functioning of the skin, such as, retinoid binding, metabolizing genes, lipid transport, and extracellular matrix. By modulating the expression of these genes a healthier and more age-resistant skin is promoted.

In an embodiment, the topical application of the ALRE is to the low neckline, and/or the face, and/or the cleavage, and/or the lips, and/or the cheeks, and/or the eyelids, and/or the hands, and/or arms, and/or body, and/or elbows of a person in need.

### Atractylodes lancea root extract

The biomass of interest is the *Atractylodes lancea (in* Chinese is called *Cangzhu*) root, preferably dried, cut, triturated, and ground root.

The biomass may be originally obtained from the wild or from cultivation, including cultivation under Organic standards.

*Atractylodes lancea* belongs to *Atractylodes* sp., which further includes *Atractylodes amurensis, Atractylodes carlinoides, Atractylodes japonica, Atractylodes koreana, Atractylodes macrocephala, Atractylodes ovata, Atractylodes lyrate, Atractylodes separata* and *Atractylodes rubra.*

The term "extract" means any compound which has been extracted from *Atractylodes lancea* root, preferably from *Atractylodes lancea* dried and triturated root. In the context of the present description, the extract is contained within the solvent mixture used for extraction. Further additives may also be used, including but not limited to additional solvents or solubilizers, antioxidants, preservatives, pH adjusters and rheology modifiers. This resulting mixture may be then directly integrated into cosmetic formulations, either as in liquid form or dried to obtain a solid extract.

The term "extraction", for purposes of this invention, means treatment of the biomass with a liquid or exposure of the biomass thereto. Compounds are retrieved from the solid biomass via solubilization in the liquid phase. In particular, compounds having bioactive properties are of special interest.

The extraction of compounds, such as botanical active ingredients, from vegetable matter is well known to the skilled in the art and is described, for example, in the book S.C. Mandal, V. Mandal and A.K. Das, Essentials of Botanical Extraction: Principles and Applications, Elsevier, San Diego, 2015.

Extraction processes are well-known to those having ordinary skill in the extract field (e.g., maceration, infusion, percolation, digestion, decoction, hot continuous extraction, counter current extraction, microwave assisted extraction, ultrasound extraction, supercritical fluid extraction, etc). Preferred extraction processes are maceration, decoction, infusion, hot continuous extraction, and combinations thereof.

The process for preparing the ALRE comprises the extraction of the root, preferably dried and triturated, with a solvent, and the separation of the root from the extract.

At the end of the extraction, the biomass is usually separated from the extract by processes well-known to those having ordinary skill in the extract field (e. g., decantation, filtration, press or vacuum filtration, centrifugation, etc). The extract can then be stored and used in liquid form, or subject to further processing techniques (e.g., heating, cooling, evaporation, concentration, pasteurization, spray-drying, lyophilization, fractionation, etc.).

Prior to the extraction process, the biomass may be subjected to fermentation or enzymatic treatments for the purposes of breaking cell walls and possibly making any bioactives present in the biomass more readily available for extraction.

In an embodiment, the ALRE is obtained by an extraction process comprising the following steps:
a) contacting *Atractylodes lancea* root with the extraction solvent forming the extraction mixture,
b) extracting the *Atractylodes lancea* root with the extraction solvent at a temperature higher than room temperature, and
c) optionally macerating the extraction mixture at room temperature.

In said extraction process, the biomass is put into intimate contact with a liquid to form a slurry, wherein the liquid is the extraction solvent. The content of the liquid is comprised usually between about 50 wt.% and about 99.5 wt.%, preferably between about 90 wt.% and about 99.5 wt.%. The solvent is usually selected from water, alcohols, polyalcohols such as glycols and glycerin, oils, and mixtures thereof. In an embodiment, the solvent is selected from water, ethanol, glycerin, 1,3-propanediol, propylene glycol, butylene glycol and mixtures thereof. In a preferred embodiment, the solvent is selected from ethanol, water, 1,3-propanediol, butylene glycol, and mixtures thereof. In a more preferred embodiment, the solvent is selected from butylene glycol, 1,3-propanediol, mixtures of ethanol water, and mixtures of butylene glycol and water.

The extract obtained with this process may contain one or more metabolites of *Atractylodes* sp., including a series of sesquiterpenoids, monoterpenes, polyacetylenes, phenolic acids, and steroids.

As disclosed in Wagner et al., Rhizoma Atractylodis lanceae Cangzhu, in: Wagner, H., Bauer, R., Melchart, D., Xiao, PG., Staudinger, A. (eds), Chromatographic Fingerprint Analysis of Herbal Medicines, 2011, Springer, Vienna, pages 691-706, main constituents of the ALRE are monoterpenes (e.g., borneol, 3-carene, *p*-cymene, α-/β-phellandrene, α-terpinene), sesquiterpenes (e.g., atractylon, elemol, α-bisabolol, muurolene, valencene, δ-cadinene, "atractylol", β-eudesmol, hinesol, 3β-acetoxyatractylon, 3β-hydroxyatractylon, atractylenolide I, II and III), phenolic constituents (thymol, carvacrol, o-cresol, *p-*cresol), *n*-dodecanol, sesquiterpene glycosides (guaiane- and eudesmane-type), triterpenes, sterols, acetylenic compounds (e.g., atractylodin, atractylodinol, acetylatractylodinol, 1Z-derivatives, (3Z,5E,11E)-tridecatriene-7,9-diyne-1,2-diyl diacetate, (4*E*,6*E*,12*E*)-tetradecatriene-8,10-diyne-1,3-diyl diacetate, threo-1-(2-furyl)-(7*E*)-nonene-3,5-diyne-1,2-diyl diacetate, (3*Z*,5*E*,11*E*)-tridecatriene-7,9-diynyl-1-O-(*E*)-ferulate), 2-[(2*E*)-3,7-dimethyl-2,6-octadienyl]-6-methyl-2,5-cyclohexadiene-1,4-dione (atractyloquinone), atractylochromene, atractylohydroquinone, monosaccharides (arabinose, galactose, glucose), furanocoumarin (osthol), and polysaccharides (actractans A,B,C).

In an embodiment, the ALRE is titrated in atractylodin and in the total phenolic content.

In an embodiment, the ALRE is in the form of a liquid extract, which usually comprises between 0.5 wt% and 5 wt% of dry matter. In a preferred embodiment, the ALRE in the form of a liquid extract comprises between 50 and 300 ppm, preferably between 50 and 250 ppm, more preferably between 50 and 200 ppm, and yet more preferably between 50 and 150 ppm of atractylodin, and between 100 and 500 ppm, preferably between 100 and 475 ppm, and more preferably between 120 and 450 ppm of the total phenolic content.

In an embodiment, the ALRE is in the form of a solid extract. In a preferred embodiment, the ALRE in the form of a solid extract comprises between 1000 and 15000 ppm, preferably between 2000 and 13000 ppm, more preferably between 3000 and 12000 ppm, and more preferably between 3500 and 11500 ppm of atractylodin, and between 5000 and 30000 ppm, preferably 6000 and 29000 ppm, and more preferably between 8000 and 28500 ppm of the total phenolic content.

The determination of dry matter is a routine analysis performed by the skilled person in the art. Dry matter may be determined, for example, by weighing a sample of the liquid extract in a covered, flat, aluminium dish and dry to constant weight at 100 °C, preferably in an oven fitted with controlled ventilation.

### Cosmetic compositions

In an embodiment of the use of the invention, the ALRE is present in a cosmetic composition.

In an embodiment, the ALRE is incorporated as a solid extract to said cosmetic composition.

In an embodiment, the ALRE is incorporated as a liquid extract to said cosmetic composition.

The amount of the ALRE in a cosmetic composition for the use of the invention depends on the desired effect and must be in a sufficient quantity for obtaining a skin anti-aging effect.

In an embodiment the cosmetic composition comprises the ALRE and a cosmetically acceptable component.

As used herein, "cosmetically-acceptable" means that the product(s) or compound(s) which the term describes are suitable for use in contact with tissues (e.g., the skin) without undue toxicity, incompatibility, instability, irritation, allergic response, and the like.

In an embodiment, the cosmetic composition comprises between 0.001 wt% and 1.2 wt% of the ALRE, expressed as dry matter, and a cosmetically acceptable component up to 100 wt%.

The composition preferably comprises between 0.005 wt% and 0.85 wt% of the ALRE, expressed as dry matter, more preferably between 0.008 wt% and 0.65 wt%, more preferably between 0.01 wt% and 0.12 wt%, and yet more preferably between 0.02 wt% and 0.06 wt%, and a cosmetically acceptable component up to 100 wt%.

In the case of using a solution of the ALRE, the skilled person in the art can determine routinely the amount of solution needed to obtain the required amount of the ALRE calculated as dry matter.

The cosmetic composition comprises a cosmetically acceptable component, which in the context of the invention, is selected from a cosmetically acceptable vehicle, a cosmetic ingredient, and mixtures thereof.

A cosmetically acceptable vehicle (or carrier) is a vehicle in which the cosmetic ingredients are dissolved, emulsified, dispersed, or suspended. Said cosmetically acceptable vehicle is usually selected from water, a water-miscible non-aqueous vehicle, such as ethanol or isopropanol or glycols, and a water-immiscible non-aqueous vehicle, such as, for example, vegetable oil, fatty esters, medium chain triglycerides, or alkanes. Preferably the cosmetic composition includes water as a vehicle.

In an embodiment, the cosmetic composition contains at least one additional cosmetic ingredient, such as, for example, cosmetic active ingredients, auxiliary products and additional additives.

In an embodiment, the additional cosmetic ingredient can be selected from: anti-inflammatory agents, anti-pruriginous agents, keratolytic agents, anti-seborrheic agents, antidandruff agents, the agents modulating the differentiation, proliferation or pigmentation of the skin and agents accelerating penetration, desquamating agents, depigmenting or pro-pigmenting agents, tightening agents, muscle relaxants, antipollution and/or anti-free radical agents, slimming agents, anticellulite agents, agents acting on the microcirculation, agents acting on the energy metabolism of the cells, cleaning agents, hair conditioning agents, hair styling agents, hair growth promoters, sunscreen and/or sunblock compounds, make-up agents, detergents, emulsifiers, emollients, antiseptic agents, deodorant actives, dermatologically acceptable carriers, surfactants, abrasives, absorbents, colorants, essential oils, cosmetic astringents, anti-acne agents, anti-caking agents, anti-foaming agents, antioxidants, binders, biological additives, enzymes, enzymatic inhibitors, enzyme-inducing agents, coenzymes, plant extracts, plant derivatives, plant tissue extracts, plant seed extracts, plant oils, botanicals, botanical extracts, ceramides, peptides, buffering agents, bulking agents, chelating agents, chemical additives, colorants, cosmetic biocides, denaturants, astringents compounds, film formers, quaternary derivatives, agents increasing the substantivity, opacifying agents, pH adjusters, pH regulators, propellants, reducing agents, sequestrants, skin bleaching and lightening agents, skin tanning agents, skin-conditioning agents, skin soothing and/or healing agents, skin treating agents, thickeners, vitamins and derivatives thereof, peeling agents, moisturizing agents, lignans, UV absorbers, suspending agents, viscosity modifiers, pigments, dyes, non-volatile solvents, diluents, pearlescent aids, foam boosters, water-soluble sunscreen, antiperspirant, depilatory, fat soluble sunscreen, skin restructuring agent, emollient, fillers, minerals, anti-mycobacterial agents, anti-allergenic agents, anti-irritants, staining agents, hypopigmenting agents, preservatives, photostabilizing agents and their mixture.

In an embodiment, the additional cosmetic ingredient is preferably selected from: surfactants (emulsifiers), lipid compounds, emollients, factors of consistency, thickening agents, stabilizers, preserving agents, essential oils, fragrances, colorants, silicone compounds, clays, fats, waxes, lecithins, phospholipids, UV sun protection factors, film-forming agents, self-tanners, firming agents, cosmetic actives, and mixtures thereof.

In a preferred embodiment, the cosmetic composition contains an additional cosmetically acceptable ingredient selected from the group consisting of surfactants (emulsifiers), lipid, emollient compounds, consistency factors, thickening agents, preservatives, cosmetic actives, and mixtures thereof.

The physical form of the cosmetic composition for the use according to the invention is not important. Said composition may be made into a wide variety of product types that include but are not limited to solid and liquid compositions. Usually, the cosmetic composition is in form of solutions, lotions, creams, gels, sticks, sprays, ointments, cleansing liquid washes and solid bars, shampoos, pastes, powders, foams, mousses, milks, emulsions, dispersions, suspensions, or wipes.

The topical compositions useful in the present invention may be formulated as solutions. Solutions may preferably include an aqueous solvent (e.g., from about 75 wt% to about 95 wt% or from about 75 wt% to about 85 wt% of a cosmetically acceptable aqueous solvent). More preferably, such compositions may contain about 30 wt% solvent, although this may vary dependent upon the formulation. Such solvents may include ethanol, propylene glycol, pentylene glycol, hexanediol, propanediol, glycerin, mixtures thereof and the like. In an embodiment, the topical composition useful in the present invention may be formulated as a solution containing an emollient. Such composition preferably contains from about 2 wt% to about 50 wt% of an emollient. As used herein, "emollients" refer to materials used for the prevention or relief of dryness, as well as for the protection of the skin.

A lotion may be made from a solution. Lotions typically contain from about 1 wt% to about 50 wt% of an emollient and from about 50 wt% to about 90 wt% of water.

Another type of product may be a cream. A cream typically comprises from about 5 wt% to about 50 wt% of an emollient and from about 45 wt% to about 85 wt% of water.

Another type of product may be an ointment. An ointment may be constituted of a simple base of vegetable oils or semi-solid hydrocarbons. An ointment may contain from about 2 wt% to about 100 wt% of an emollient, and from about 0.1 wt% to about 5 wt% of a thickening agent.

The topical compositions useful in the present invention may also be preferably formulated as emulsions. In an embodiment, the cosmetic composition contains water and a lipophilic phase and is presented in the form of emulsion or dispersion, such as, for example, the oil-in-water (O/W), water-in-oil (W/O) type, multiple emulsion (W/O/W), or PIT-type emulsion, or microemulsion. If the carrier is an emulsion, from about 1 wt% to about 10 wt% of the carrier should be made up one or more emulsifiers. Emulsifiers may be non-ionic, anionic, cationic, or amphoteric.

The topical composition useful in the present invention may be formulated as a gel. Suitable gelling agents for aqueous gels include, but are not limited to, natural gums, acrylic acid and acrylate polymers and copolymers, and cellulose derivatives (e.g., hydroxymethyl cellulose and hydroxypropyl cellulose). Suitable gelling agents for oils (such as vegetable oils, esters) include, but are not limited to, waxes, modified silicas, cellulose derivatives, polyamides, polyurethanes, and L-glutamic acid derivatives. Such gels typically comprise between about 0.1 wt% and 20 wt% of such gelling agents.

The topical composition useful in the present invention may also be formulated into a solid formulation (e.g., a wax-based stick, mascara, soap bar composition, powder, a wipe containing powder, or cosmetotextile).

In a preferred embodiment, the cosmetic composition comprises:
a) between 0.001 wt% and 1.2 wt% of the ALRE, expressed as dry matter,
b) between 5 wt% and 50 wt% of at least one additional cosmetically acceptable ingredient, and
c) between 49 wt% and 94 wt% of a cosmetically acceptable vehicle,
where the percentages of the components are adjusted so that the balance is 100%.

In an embodiment, the content of the ALRE is comprised between 0.005 wt% and 0.85 wt%, expressed as dry matter, preferably between 0.008 wt% and 0.65 wt%, more preferably between 0.01 wt% and 0.12 wt%, and yet more preferably between 0.02 wt% and 0.06 wt%.

In an embodiment, the content of the additional cosmetic ingredient is preferably between 10 wt% and 45 wt%, more preferably between 15 wt% and 35 wt%, and even more preferably between 20 wt% and 25 wt%.

In an embodiment the content of the vehicle is preferably between 50 wt% and 90 wt%, more preferably between 55 wt% and 87 wt%, and even more preferably between 65 wt% and 85 wt%.

The cosmetic composition useful for the use of the present invention are generally prepared by conventional methods such as are known in the art of making topical compositions. Such methods typically can involve mixing of the ingredients in one or more steps to a relatively uniform state, with or without heating, cooling, application of vacuum, application of high shear, ultrasounds, and the like.

### Surfactants (emulsifiers)

In an embodiment, the cosmetic composition can include surfactants (emulsifiers) to facilitate solution, emulsion, dispersion, or suspension of cosmetic components.

Surfactants can be anionic, non-ionic, cationic and/or amphoteric.

The content of surfactants is usually comprised between 1 wt% and 30 wt%, preferably between 2 wt% and 20 wt%, more preferably between 3 wt% and 10 wt%, and still more preferably between 4 wt% and 8 wt%.

Typical examples of anionic surfactants are, for example, soaps, sulfonated alkanes, sulfonated olefins, alkyl sulfates, fatty alcohol ether sulfates, glycerol ether sulfates, fatty acid ether sulfates, mono- and dialkyl sulfosuccinates, mono- and dialkyl sulfosuccinamates, ether carboxylic acids and their salts, fatty acid isethionates, fatty acid sarcosinates, fatty acid taurides, N-acylamino acids.

Typical examples of non-ionic surfactants are, for example, polyalkoxylated fatty alcohols, polyalkoxylated fatty acids, polyalkoxylated fatty acid amides, polyalkoxylated fatty amines, alkoxylated triglycerides, mixed ethers, alkylpolyglycosides, *N*-alkyl sorbitan esters, fatty acid esters polyethoxylated sorbitan, and amine oxides.

Typical examples of cationic surfactants are, for example, quaternary ammonium compounds, and quaternized salts of esters of trialkanolamines and fatty acids, for example Esterquats.

Typical examples of amphoteric surfactants are, for example, alkylbetaines, alkylamidobetaines, aminopropionates, aminoglycinates, imidazolinium betaines and sulfobetaines.

The aforementioned surfactants are exclusively known compounds, the structure and preparation of which are well known to those skilled in the art, and can be found, for example, in X. Domingo's book, A guide to the surfactants world, Edicions Proa, Barcelona, 1995.

### Lipid components and emollients

The cosmetic composition usually contains additional lipid compounds and emollients to optimize their organoleptic and dermatological properties.

The lipid and emollient compounds are usually contained in a total amount of between 1 wt% and 90 wt%, preferably between 5 wt% and 25 wt%, and more preferably between 5 wt% and 15 wt%.

Suitable lipid compounds are Guerbet alcohols based on fatty alcohols with 6 to 18 carbon atoms, preferably with 8 to 10 carbon atoms (such as the product Eutanol^{®} G from the BASF company), linear fatty acid esters C₆₋₂₂ with linear C₆₋₂₂ alcohols, C₆₋₁₃ branched carboxylic acid esters with C₆₋₂₂ linear alcohols, such as myristyl myristate, carbon palmitate myristyl, myristyl stearate, behenyl stearate, behenyl isostearate, behenyl oleate, behenyl behenate, behenyl erucate, or erucyl myristate.

Furthermore, C₆₋₂₂ linear fatty acid esters with branched alcohols are suitable, especially 2-ethylhexanol (for example the product Cetiol^{®} 868 from the BASF company); C₁₈₋₃₈ alkylhydroxycarboxylic acid esters with linear or branched C₆₋₂₂ fatty alcohols; linear and/or branched fatty acid esters with polyhydric alcohols, and/or Guerbet alcohols; triglycerides based on fatty acids with 6 to 10 carbon atoms (for example the product Mirytol^{®} 318 from the company BASF); liquid mixtures of mono/di/triglycerides based on fatty acids with 6 to 18 carbon atoms; esters of fatty alcohols with 6 to 22 carbon atoms, and/or Guerbet alcohols with aromatic carboxylic acids, especially benzoic acid; esters of dicarboxylic acids with 2 to 12 carbon atoms with linear or branched alcohols of 1 to 22 carbon atoms, or polyols with 2 to 10 carbon atoms and 2 to 6 hydroxyl groups; vegetable oils; branched primary alcohols; substituted cyclohexanes; linear and branched C₆₋₂₂ fatty alcohol carbonates, such as, for example, dicaprylyl carbonates (for example the product Cetiol^{®} CC from the BASF company); symmetric or asymmetric linear or branched dialkyl ethers with 6 to 22 carbon atoms per alkyl group, such as dicaprylyl ethers (for example the product Cetiol^{®} OE from the BASF company); aliphatic or naphthenic hydrocarbons, such as squalane, squalene or dialkylcyclohexanes, and mixtures thereof.

### Consistency factors and thickeners

Consistency factors and thickeners are generally used in the cosmetic composition to adjust the viscosity and rheological behaviour.

Among the consistency factors come into consideration, firstly, fatty alcohols with a chain of 12 to 22 carbon atoms, and preferably of 16 to 18 carbon atoms, and furthermore partial glycerides, fatty acids or hydroxylated fatty acids.

Suitable thickening agents are, for example, hydrophilic silicic anhydride (such as the Aerosil^{®} products from the Evonik company); polysaccharides, especially xanthan gum, guar gum, tara gum, agar-agar, alginates and tyloses, carrageenan, sclerotium gum, carboxymethylcellulose and hydroxyethylcellulose, as well as polyethylene glycol mono- and higher molecular weight fatty acid diesters; polyacrylates (for example Carbopol^{®} and Pemulen^{®} types from the Lubrizol company; Synthalene^{®} from the Sigma company, Keltrol^{®} types from the CP Kelco company; Sepigel^{®} and Simulgel^{®} types from the Seppic company; Salcare^{®} types from the company Allied Colloids company), polyacrylamides, polyvinyl alcohol, and polyvinylpyrrolidone.

In order to improve the flowability of the cosmetic composition, it is also possible to use for example, ethanol, isopropyl alcohol, or polyols. The polyols which come into consideration here preferably have from 2 to 15 carbon atoms and at least two hydroxyl groups. The polyols can also contain other functional groups, especially amino groups, or be modified with nitrogen. Typical examples are glycerol; alkylene glycols, such as, for example, ethylene glycol, diethylene glycol, propylene glycol, butylene glycol, hexylene glycol, as well as polyethylene glycols with an average molecular weight of 100 to 1,000 daltons; technical oligoglycerol mixtures with a degree of self-condensation of 1.5 to 10, for example technical diglycerol mixtures with a diglycerol content of 40 wt% to 50 wt%; methylol compounds, such as especially trimethylolmethane, trimethylolpropane, trimethylolbutane, pentaerythritol and dipentaerythritol; alkylglycosides with a chain of 1 to 8 carbon atoms in the alkyl moiety; C₅₋₁₂ sugar alcohols, such as sorbitol or mannitol; sugars with 5 to 12 carbon atoms, such as glucose or sucrose; amino sugars, such as glucamine; dialcoholamines, such as diethanolamine or 2-amino-1,3-propanediol.

In an embodiment, glycerol, propylene glycol, and their mixtures are used as solvents in the cosmetic composition.

### Preservative agents

Suitable preservatives to be used in the cosmetic composition include, by way of example, phenoxyethanol, 3-(4-chlorophenoxy)-1,2-propanediol (chlorphenesin), pentanediol or sorbic acid, and the classes of additional substances indicated in Annex V of the Regulation (EC) No 1223/2009 of the European Parliament and of the Council, as well as preservation boosters such as levulinic acid, 1,2-hexanediol or ethylhexylglycerin.

### Cosmetic method for skin anti-aging

Another aspect of the present invention is a cosmetic method for skin anti-aging comprising the step of topically applying to skin the ALRE or a cosmetic composition comprising the ALRE, wherein the ALRE is the only anti-aging active.

In an embodiment, the ALRE is obtained according to the method disclosed above.

In an embodiment, the method of the invention comprises the step of topically applying the ALRE or a cosmetic composition comprising the ALRE to, and/or the low neckline, and/or the face, and/or the cleavage, and/or the lips, and/or the cheeks, and/or the eyelids, and/or the hands, and/or arms, and/or body, and/or elbows.

In an embodiment, the ALRE or the cosmetic composition comprising the ALRE is applied topically to the desired area of the body at least once daily, preferably between 1 to 3 times daily, and more preferably, 1 to 5 times daily.

In an embodiment, the ALRE is applied topically to the desired area of the body for at least 2 weeks, preferably at least 4 weeks, more preferably at least 8 weeks, and more preferably at least 12 weeks.

The present invention may be defined according to the following embodiments:
1.- Cosmetic use of an *Atractylodes lancea* root extract (ALRE) for skin anti-aging by topical application, wherein said extract is the only anti-aging active.
2.- Cosmetic use according to embodiment 1, wherein the use of the ALRE is selected from for stimulating of collagen synthesis, for not inducing MMP activity, for smoothing wrinkles, for reducing the oxidative stress, and for modulating of gene expression to influence the expression of genes related to the health and proper functioning of the skin, such as, retinoid binding genes, metabolizing genes, lipid transport genes, and extracellular matrix genes.
3.- Cosmetic use according to embodiment 2, wherein the use of the ALRE is for stimulating collagen synthesis.
4.- Cosmetic use according to embodiment 2, wherein the use of the ALRE is for not inducing MMP activity.
5.- Cosmetic use according to embodiment 2, wherein the use of the ALRE is for smoothing wrinkles.
6.- Cosmetic use according to embodiment 2, wherein the use of the ALRE is for modulating of gene expression to influence the expression of genes related to the health and proper functioning of the skin, such as, retinoid binding genes, metabolizing genes, lipid transport genes, and extracellular matrix genes.
7.- Cosmetic use according to any one of embodiments 1 to 6, wherein the topical application of the ALRE is to the low neckline, and/or the face, and/or the cleavage, and/or the lips, and/or the cheeks, and/or the eyelids, and/or the hands, and/or arms, and/or body, and/or elbows, of a person in need.
8.- Cosmetic use according to any one of embodiments 1 to 7, wherein the biomass of interest is the *Atractylodes lancea* root dried, cut, triturated, and ground root.
9.- Cosmetic use according to any one of embodiments 1 to 8, wherein the ALRE is obtained by an extraction process comprising the following steps:
   a) contacting *Atractylodes lancea* root with the extraction solvent forming the extraction mixture,
   b) extracting the *Atractylodes lancea* root with the extraction solvent at a temperature higher than room temperature, and optionally
   c) macerating the extraction mixture at room temperature.
10.- Cosmetic use according to embodiment 9, wherein the solvent is selected from water, alcohols, polyalcohols such as glycols and glycerin, oils, and mixtures thereof.
11.- Cosmetic use according to embodiment 10, wherein the solvent is selected from water, ethanol, glycerin, 1,3-propanediol, propylene glycol, butylene glycol and mixtures thereof.
12.- Cosmetic use according to embodiment 11, wherein the solvent is selected from ethanol, water, 1,3-propanediol, butylene glycol, and mixtures thereof.
13.- Cosmetic use according to embodiment 12, wherein the solvent is selected from butylene glycol, 1,3-propanediol, mixtures of ethanol water, and mixtures of butylene glycol and water.
14.- Cosmetic use according to any one of embodiments 1 to 13, wherein the ALRE is in the form of a solid extract.
15.- Cosmetic use according to embodiment 14, wherein the solid extract comprises between 1000 and 15000 ppm, preferably between 2000 and 13000 ppm, more preferably between 3000 and 12000 ppm, and more preferably between 3500 and 11500 ppm of atractylodin, and between 5000 and 30000 ppm, preferably 6000 and 29000 ppm, and more preferably between 8000 and 28500 ppm of the total phenolic content.
16.- Cosmetic use according to any one of embodiments 1 to 13, wherein the ALRE is in the form of a liquid extract.
17.- Cosmetic use according to embodiment 16, wherein the liquid extract comprises between 0.5 wt% and 5 wt% of dry matter.
18.- Cosmetic use according to any one of embodiments 16 or 17, wherein the ALRE in the form of a liquid extract comprises between 50 and 300 ppm, preferably between 50 and 250 ppm, more preferably between 50 and 200 ppm, and yet more preferably between 50 and 150 ppm of atractylodin, and between 100 and 500 ppm, preferably between 100 and 475 ppm, and more preferably between 120 and 450 ppm of the total phenolic content.
19.- Cosmetic use according to any one of embodiments 1 to 18, wherein the ALRE is present in a cosmetic composition.
20.- Cosmetic use according to embodiment 19, wherein the ALRE is incorporated as a solid extract to the cosmetic composition.
21.- Cosmetic use according to embodiment 19, wherein the ALRE is incorporated as a liquid extract to the cosmetic composition.
22.- Cosmetic use according to any one of embodiments 19 to 21, wherein the cosmetic composition comprises the ALRE and a cosmetically acceptable component.
23.- Cosmetic use according to embodiment 22, wherein the cosmetic composition comprises between 0.001 wt% and 1 wt% of the ALRE, expressed as dry matter, and a cosmetically acceptable component up to 100 wt%.
24.- Cosmetic use according to embodiment 23, wherein the cosmetic composition comprises between 0.005 wt% and 0.85 wt% of the ALRE, expressed as dry matter, preferably between 0.008 wt% and 0.65 wt%, more preferably between 0.01 wt% and 0.12 wt%, and yet more preferably between 0.02 wt% and 0.06 wt%.
25.- Cosmetic use according to any one of embodiments 22 to 24, wherein the cosmetically acceptable component is selected from a cosmetically acceptable vehicle, a cosmetic ingredient, and mixtures thereof.
26.- Cosmetic use according to embodiment 25, wherein the cosmetically acceptable vehicle is selected from water, a water-miscible non-aqueous vehicle, and a water-immiscible non-aqueous vehicle.
27.- Cosmetic use according to embodiment 26, wherein the cosmetically acceptable vehicle is water.
28.- Cosmetic use according to any one of embodiments 25 to 27, wherein the cosmetically acceptable ingredient is selected from: surfactants (emulsifiers), lipid compounds, emollients, factors of consistency, thickening agents, stabilizers, preserving agents, essences, colorants, silicone compounds, fats, waxes, lecithins, phospholipids, UV sun protection factors, film-forming agents, self-tanners, firming agents, cosmetic actives, and mixtures thereof.
29.- Cosmetic use according to any one of embodiments 19 to 28, wherein the cosmetic composition is in form of solutions, lotions, creams, gels, sticks, sprays, ointments, cleansing liquid washes and solid bars, shampoos, pastes, powders, foams, mousses, milks, emulsions, dispersions, suspensions, or wipes.
30.- Cosmetic use according to any one of embodiments 19 to 29, wherein the cosmetic composition comprises:
   a) between 0.001 wt% and 1.2 wt% of the ALRE, expressed as dry matter, preferably between 0.005 wt% and 0.85 wt% of the ALRE, more preferably between 0.008 wt% and 0.65 wt%, more preferably between 0.01 wt% and 0.12 wt%, and yet more preferably between 0.02 wt% and 0.06 wt%
   b) between 5 wt% and 50 wt% of at least one additional cosmetically acceptable ingredient, is preferably between 10 wt% and 45 wt%, more preferably between 15 wt% and 35 wt%, and even more preferably between 20 wt% and 25 wt%, and
   c) between 49 wt% and 94 wt% of a cosmetically acceptable vehicle, preferably between 50 wt% and 90 wt%, more preferably between 55 wt% and 87 wt%, and even more preferably between 65 wt% and 85 wt%,
   where the percentages of the components are adjusted so that the balance is 100%.
31.- Cosmetic method for skin anti-aging comprising the step of topically applying to skin the ALRE according to any one of embodiments 14 to 18, or a cosmetic composition according to any one of embodiments 19 to 30, wherein the ALRE is the only anti-aging active.
32.- Cosmetic method for skin anti-aging according to embodiment 31 comprising the step of topically applying the ALRE or the cosmetic composition to the low neckline, and/or the face, and/or the cleavage, and/or the lips, and/or the cheeks, and/or the eyelids, and/or the hands.
33.- Cosmetic method according to embodiment 31 or 32, wherein the ALRE or the cosmetic composition comprising the ALRE is applied topically to the desired area of the body at least once daily, preferably between 1 to 3 times daily, and more preferably, 1 to 5 times daily.
34.- Cosmetic method according to any one of embodiments 31 to 33, wherein the ALRE or the cosmetic composition comprising the ALRE is applied topically to the desired area of the body for at least 2 weeks, preferably at least 4 weeks, more preferably at least 8 weeks, and more preferably at least 12 weeks.

### EXAM PLES

The content of atractylodin was determined by HPLC, taking as a reference a commercially available sample of the compound. The HPLC method is disclosed for example in Wagner *et al., op. cit.*

The total phenolic content (TPC) was analysed according to the method disclosed in, for example, AOAC Official Methods Analysis, 2015, SMPR 2015.009.

The geographic origin of the *Atractylodes lancea* roots used in the preparative examples, in *in vitro* and *in vivo* tests is China.

### Preparative examples:

### 1.- Preparation of an Atractylodes lancea root extract using ethanol/water

*Atractylodes lancea* dried triturated root (21.96 g) wase placed in a reflux system along with 193.03 g of ethanol and 83.09 g of water. The mixture was refluxed for 3 h, the liquid was decanted (260.23 g) and to the remaining biomass additional ethanol (194.11 g) and water (82.21 g) were added. This mixture was refluxed again for 3 h. The liquid was decanted (270.28 g) and mixed with the liquid from the first reflux. Solvents were then evaporated under vacuum to yield a soft extract.

This extract contained 3600 ppm of atractylodin and 8300 ppm Total Phenolic Content (TPC).

### 2.- Preparation of an Atractylodes lancea root extract using 1,3-propanediol

To 1,3-propanediol (95 g), *Atractylodes lancea* dried triturated root (10 g) was added and temperature was maintained at 70 °C for 2 h. The mixture was allowed to cool to room temperature, and it was then macerated for 16 h. The spent biomass was pressed, and the liquid was recovered and heated to 100 °C. The liquid was then clarified by filtration with the aid of diatomaceous earths.

The resulting product with a dry residue of 1.52% contained 104 ppm of atractylodin and 347 ppm of Total Phenolic Content (TPC).

### 3.- Preparation of an Atractylodes lancea root extract using 1,3-propanediol/water

To a solvent mixture of 1,3-propanediol (108 g) and water (12 g) was added sodium hydroxide (20% solution) to adjust the pH to 4.3-4.6 with and the mixture was heated to 90-100 °C. *Atractylodes lancea* dried triturated root (10 g) was added to the mixture and the temperature was maintained at 70 °C for 2 h. The mixture was allowed to cool to room temperature, and it was then macerated for 16 h. The spent biomass was pressed, and the liquid was recovered and heated to 100 °C. The liquid was then clarified by filtration with the aid of diatomaceous earths.

The resulting product with a dry residue of 2.07 % contained 103 ppm of atractylodin and 430 ppm of Total Phenolic Content (TPC).

### 4.- Preparation of an Atractylodes lancea root extract using butylene glycol

To butylene glycol (120 g), *Atractylodes lancea* dried triturated root (10 g) was added and temperature was maintained at 70 °C for 2 h. The mixture was allowed to cool to room temperature, and it was then macerated for 16 h. The spent biomass was pressed, and the liquid was recovered and heated to 100 °C. The liquid was then clarified by filtration with the aid of diatomaceous earths.

The resulting product with a dry residue of 0.79% contained 61 ppm of atractylodin and 160 ppm of Total Phenolic Content (TPC).

### 5.- Preparation of an Atractylodes lancea root extract using butylene glycol/water

To a solvent mixture of butylene glycol (108 g) and water (12 g), citric acid (0.2 g) was added. The pH was adjusted to 4.3-4.6 with sodium hydroxide solution (20%) and the mixture was heated to 90-100 °C. *Atractylodes lancea* dried triturated root (10 g) was added to the mixture and the temperature was maintained at 70 °C for 2 h. The mixture was allowed to cool to room temperature, and it was then macerated for 16 h. The spent biomass was pressed, and the liquid was recovered and heated to 100 °C. The liquid was then clarified by filtration with the aid of diatomaceous earths.

The resulting product with a dry residue of 1.18 % contained 130 ppm of atractylodin and 333 ppm of Total Phenolic Content (TPC).

The content of atractylodin and Total Phenolic Content, expressed both in ppm, obtained with different extraction procedures, is summarized in Table I:

**TABLE I**

| Preparative Example | Form/Dry residue | Atractylodin | Total Phenolic Content |
|---|---|---|---|
| 1 | Solid/100% | 3600 | 8300 |
| 2 | Liquid/1.52% | 104 | 347 |
| 3 | Liquid/2.07% | 103 | 430 |
| 4 | Liquid/0.79% | 61 | 160 |
| 5 | Liquid/1.18% | 130 | 333 |

### Example 1: Irritation test

The skin irritation and inflammation due to the topical use of retinoids has been linked to the activation of the transient receptor potential vanilloid 1 (TRPV1) channel, a non- selective cation channel within the TRPs family, as disclosed in Yin et al., Retinoids activate the irritant receptor TRPV1 and produce sensory hypersensitivity, J. Clin. Invest., 2013, 123(9),3941-3951.

In this TRPV1 test, compositions comprising different amounts of the *A. lancea* root extract (ALRE) were compared with retinol and tested on Human Embryonic Kidney (HEK) cells stably expressing this TRPV1 channel.

Cytosolic calcium influx was monitored using Fluo4-NW (Thermo Fisher Scientific) mix dye following manufacturer's instructions.

Activation of the TRPV1 receptor causes a Ca⁺² influx, and a direct relationship between fluorescence and TRPV1 receptor response can be established.

Fluorescence signal (AFU) was measured after excitation at 485 nm and emission at 520 nm using ClarioStar Plus microplate reader from BMG LABTECH.

The effect of tested compounds on intracellular Ca⁺² influx was studied 48 hours after cell seeding. The medium of TRPV1-overexpressing HEK cells was replaced by 90 µL/well of Fluo4-NW dye solution in assay buffer containing probenecid. Then, 10 µL/well of each test substance solution was added and incubated together with the dye during 60 minutes at 37 °C in a cell incubator or added directly during the measurement of the plate on cycle 3 (Figure 1).

The ALRE was assayed at concentrations of 0.20, 0.10 and 0.05 mg/mL while retinol and all-trans retinoic acid (ATRA) at 100, 50 and 10 µM. Vehicle was DMSO at 0.20, 0.10 and 0.05 % (v/v) or assay buffer.

After 1 hour incubation at 37 °C, the plate was kept for 15 minutes at room temperature inside the fluorescence plate reader. After that, the program started with a stop in cycle 3 to add test compounds and capsazepine, 0.2% Triton X-100 (maximal signal control for dye loading) and assay buffer (minimal signal) and the fluorescence signal was recorded for 7 additional cycles. Then, 10 µL/well of agonist capsaicin at 1 µM was automatically added by injector in all wells (except maximal and minimal signal controls). The signal was recorded until cycle 20. Each condition was performed in triplicate (n=3) at least in 3 independent experiments (N=3). The response to the assay buffer (AB) without capsaicin injection was considered 0 % response, while response to 1 µM capsaicin as 100%.

In Figure 1, it can be observed that retinol triggered a significant rise in intracellular calcium at concentrations of 100 µM (0.002865 mg/ml) and 50 µM (0.014325 mg/ml). In contrast, no discernible signal increase was observed for the ALRE at any of the tested concentrations, with 0.20 mg/ml being the highest concentration tested.

Therefore, the ALRE does not activate and sensitize the nociceptors, as done by retinoids, which are susceptible to induce pain and neurogenic inflammation.

### Example 2: Gene expression by transcriptome analysis

To better understand the cellular pathways and processes that were modulated by the ALRE in human dermal fibroblasts, and its relevance with respect to retinol, a comprehensive and comparative assessment of gene expression by transcriptome analysis was conducted.

Three biological triplicates of Human Dermal Fibroblasts (HDF; ZenBio Inc., Research Triangle Park, NC, USA) were stimulated for 6 h with either 10 µM retinol or 0.05 mg/ml ALRE. RNA was extracted with the TRIzol^{®} Plus RNA Purification Kit, including DNase I treatment step, according to manufacturer's instructions. RNA quality and quantity were inspected by spectrophotometric measurement using a NanoDrop^{™} One spectrophotometer (Thermo Fisher Scientific, Pittsburgh, PA, USA).

Messenger RNA was purified from total RNA using poly-T oligo-attached magnetic beads. After fragmentation, the first strand cDNA was synthesized using random hexamer primers, followed by the second strand cDNA synthesis using either dUTP for directional library or dTTP for non-directional library. For the non-directional library, it was ready after end repair, A-tailing, adapter ligation, size selection, amplification, and purification. For the directional library, it was ready after end repair, A-tailing, adapter ligation, size selection, USER enzyme digestion, amplification, and purification . The library was checked with Qubit and real-time PCR for quantification and bioanalyzer for size distribution detection. Quantified libraries were pooled and sequenced (Paired-end 150 bp) on Illumina platforms, according to effective library concentration and data amount.

Obtained data were studied by bioinformatic analysis as disclosed, for example, in Liao et al, featureCounts: an efficient general purpose program for assigning sequence reads to genomic features, Bioinformatics, 2014 ,30(7), 923-30, Mortazavi et al., Mapping and quantifying mammalian transcriptomes by RNA-Seq, Nature Methods, 2008, 5(7), 621-628, and Robinson et al., edgeR: a Bioconductor package for differential expression analysis of digital gene expression data, Bioinformatics, 2010, 26(1), 139-140.

Genes involved in the following categories were studied:
2.1.- Retinoid binding, metabolizing genes and lipid transport
2.2.- Extracellular matrix
2.3.- Inflammatory pathway

### 2.1.- Retinoid binding, metabolizing genes and lipid transport

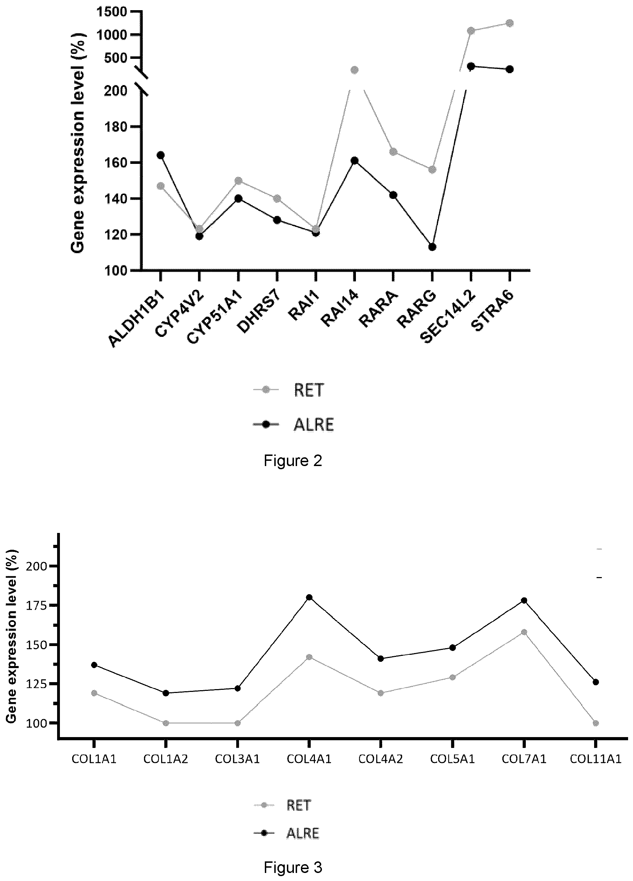

In all tissues, including the skin, the intracellular concentrations of retinoic acid are meticulously regulated. Its availability in the epidermis is governed by the expression of enzymes responsible for retinoic acid metabolism and proteins involved in its delivery and sequestration. The results of testing with HDF showed a notable upregulation of genes associated with "Retinoid binding, metabolizing genes and lipid transport" with each gene's expression level compared to untreated human fibroblasts. These upregulated genes include aldehyde dehydrogenase 1 family member B1 (ALDH1 B1), cytochrome P450 family 4 subfamily V member 2 (CYP4AV2), cytochrome P450 family 51 subfamily A member 1 (CYP51A1), dehydrogenase/reductase 7 (DHRS7), retinoic acid induced 1 (RAI1), retinoic acid induced 14 (RAI14), retinoic acid receptor alpha (RARα), retinoic acid receptor gamma (RARγ), SEC14 like lipid binding 2 (SEC14L2), and signalling receptor and transporter of retinol STRA6 (STRA6). As shown in Figure 2, many of the significantly upregulated genes exhibited similar levels of expression by the presence of retinol or the ALRE.

### 2.2.- Extracellular matrix

The extracellular matrix (ECM) within the dermal layers of the skin constitutes a multifaceted and dynamic network of macromolecules, pivotal for both structural integrity and the execution of vital functions. Predominantly, this intricate matrix comprises fibrous proteins, notably collagen, elastin, and fibronectin, alongside proteoglycans and glycosaminoglycans (GAGs). These constituents collectively confer upon the skin its tensile strength, elasticity, and moisture-retaining properties. The ECM fulfils a crucial role in upholding the skin's barrier function by actively participating in the formation of the stratum corneum, the outermost layer of the epidermis. It is noteworthy that with the progression of aging, the composition and arrangement of the ECM may undergo modifications, resulting in diminished elasticity, the emergence of wrinkle formations, and other age-related manifestations.

A comparative analysis of the impact of retinol and the ALRE on the gene expression associated with crucial ECM proteins was carried out. Both compounds exhibited comparable modulation of gene expression that contributes significantly to the composition of the dermis. In fact, the gene expression profiles were virtually indistinguishable, with the ALRE notably inducing higher expression levels of various collagen types when compared to retinol.

In Figure 3 is depicted the gene expression for seven collagen types. Type 1 Collagen, constituting 80-90% of skin collagen, bolsters skin firmness and elasticity, while Type 3 Collagen, intertwined with Type 1, enhances suppleness. Other collagen types like Type 4 and Type 7 play specialized roles in maintaining skin structure and function as well.

The gene upregulation by the ALRE significantly surpasses retinol in its impact on these seven types of collagen-related genes, indicating a superior ability to enhance the skin's ECM.

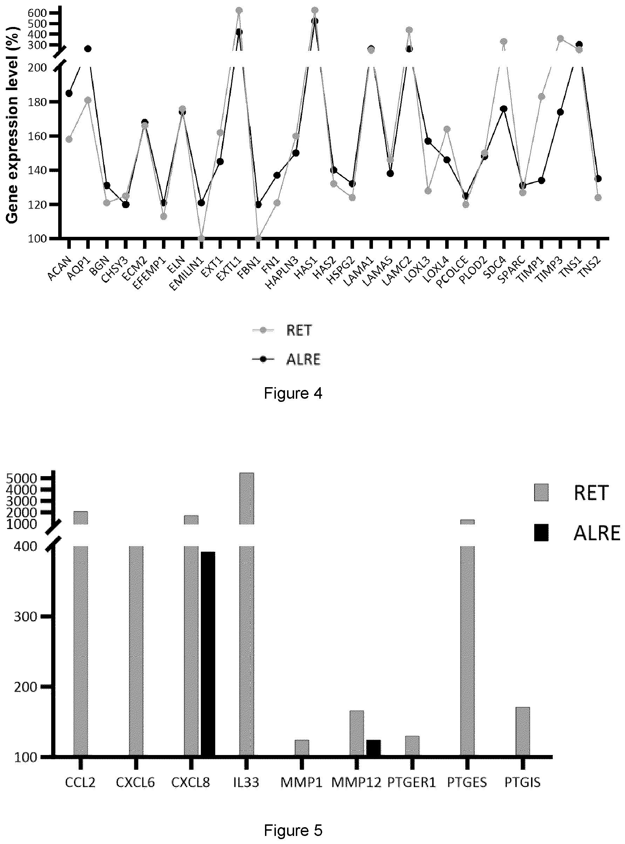

Figure 4 shows various genes associated with the ECM. Studies have emphasized the significance of Tissue Inhibitors of Metalloproteinases (TIMPs) as pivotal elements in thwarting the degradation of ECM proteins. In this context, both Retinol and the ALRE have significantly upregulated TIMP1 and TIMP3, actively contributing to the preservation of the ECM.

### 2.3.- Inflammatory pathway

Matrix metalloproteinases (MMPs) hold a significant role in some processes such as tumour invasion, inflammation, and skin aging. Both natural aging and premature aging, often triggered by UV radiation, stimulate the expression of MMPs, leading to the degradation of dermal collagen and other extracellular matrix proteins, as disclosed in Quan et al., Matrix-degrading metalloproteinases in photoaging, J. Investig. Dermatol. Symp. Proc., 2009, 14(1), 20-24. This, in turn, contributes to the development of wrinkles and sagging. In this study, an elevation in the levels of certain MMPs was observed when retinol was employed as a treatment, including MMP1 and MMP12 (Figure 5). In contrast, the use of the ALRE did not induce any increase in the expression of MMP1, which is primarily associated with collagen degradation. For the MMP12, the upregulation of gene expression due to the ALRE was comparatively lower than that observed with retinol. Consequently, the action of the ALRE is poised to support the preservation of youthful skin characteristics by attenuating the collagen-degrading effects of these enzymes.

Furthermore, exposure to UVB radiation and various other forms of skin insults induce the upregulation of inflammatory cytokines, thereby contributing to both acute and chronic skin damage, facilitated by inflammatory mediators produced by ROS, which are commonly associated with the aging process. Scientific research has confirmed that inflammation is an inherent aspect of the aging process, frequently linked to increased levels of circulating cytokines and proinflammatory markers. Despite the numerous advantages of retinol and its derivatives in dermatological applications, they are known to induce considerable localized irritation, characterized by mild erythema and the peeling of the stratum corneum of the skin. It is postulated that cytokines may play a pivotal role as significant inflammatory mediators in the development of retinoid-induced dermatitis. Among the proinflammatory cytokines, MCP-1 and IL-8 have been identified as the primary contributors to the skin irritation triggered by retinol, as disclosed in Kim et al., The mechanism of retinol-induced irritation and its application to anti-irritant development, Toxicol. Lett., 2003, 146(1), 65-73. Consequently, strategies aimed at either suppressing the production or preventing the stimulation of these cytokines serve as valuable approaches for mitigating the adverse effects associated with retinol-induced skin irritation.

In relation to the genes associated with the inflammatory pathway, the results indicate an upregulation in the gene expression of CCL2 (also known as MCP-1), CXCL6, and CXCL8 (also known as IL-8), IL33, PTGES, and PTGIS. These changes were predominantly evident only when retinol was introduced, suggesting the possibility of an undesired effect triggered by the incubation period with retinol. These findings align with prior research that has shown retinol's potential to induce proinflammatory cytokines, including CCL2 and IL-8, which are implicated in mediating the skin irritation caused by retinol, as disclosed in Kim *et al., op. cit.*, , as well as an upsurge in COX-2 expression, leading to increased PGE2 production, as disclosed in Alique et al., All-trans retinoic acid induces COX-2 and prostaglandin E2 synthesis in SH-SY5Y human neuroblastoma cells: Involvement of retinoic acid receptors and extracellular-regulated kinase 1/2. J. Neuroinflam., 2007, 4(1), 1-9. However, when fibroblasts were exposed to the ALRE, only CXCL8 (IL8) exhibited an increase in expression, albeit to a significantly lesser extent than with retinol treatment (5 times less). Furthermore, the expression of PTGES was inversely affected, showing a positive decrease when cell were treated with the ALRE.

### Example 3: Depigmentation test

The depigmenting potential of the ALRE was assessed on reconstructed human pigmented epidermis (RHPE). Said cells were specifically generated for screening depigmenting agents or skin lightening products prior to human in vivo testing, as disclosed in Hall et al., Reconstructed human pigmented skin/epidermis models achieve epidermal pigmentation through melanocore transfer, Pigment Cell Melanoma Res., 2022, 35, 425-435.

On day 9, the medium was replaced with a daily renewal, incorporating test compounds: the ALRE at concentrations of 0.2, 0.1, and 0.05 mg/ml, comparing its effect with 10 µM Retinol.

In this assessment, 100 mg/ml DMSO was used as a negative control and 250 µM Kojic acid as a positive control.

Images for melanin evaluation were captured on day 17 using Leica EZ4 W stereo microscope (Leica Microsystems, Wetzlar, Germany) and quantified as follows: all imaging processing steps were performed with the imaging software Fiji/lmageJ v 1.52n (Schindelin et al., Fiji - an Open Source platform for biological image analysis, Nat. Methods, 2012, 9(7), doi:10-1038).

Segmentation of melanin-positive areas was conducted with the machine learning tool for microscopy pixel classification Trainable Weka Segmentation (TWS) (Arganda-Carreras et al., Trainable Weka Segmentation: a machine learning tool for microscopy pixel classification, Bioinformatics, 2017, 33(15), 2424-2426), available as a plug-in within Fiji/lmageJ.

Statistical differences were determined by two-way ANOVA using R version 4.3.2 (R: A Language and Environment for Statistical Computing, n.d.). The alpha nominal level was set at 0.05 in all cases.

As it can be observed in Figure 6, kojic acid and retinol exhibited melanin reduction activities of 58% and 76%, respectively. The ALRE demonstrated a concentration-dependent effect, with significantly low values observed for the two highest concentrations: 77% for C2 (0.1 mg/ml) and 63% for C3 (0.2 mg/ml).

Consequently, the ALRE exhibits whitening properties showing retinol-like effects.

## Claims

1. Cosmetic use of an *Atractylodes lancea* root extract (ALRE) for skin anti-aging by topical application, wherein said extract is the only anti-aging active.

2. Cosmetic use according to claim 1, wherein the use of the ALRE is selected from for stimulating of collagen synthesis, for not inducing MMP activity, for smoothing wrinkles, for reducing the oxidative stress, and for modulating of gene expression to influence the expression of genes related to the health and proper functioning of the skin, such as, retinoid binding genes, metabolizing genes, lipid transport genes, and extracellular matrix genes.

3. Cosmetic use according to claim 1 or 2, wherein the topical application of the ALRE is to the low neckline, and/or the face, and/or the cleavage, and/or the lips, and/or the cheeks, and/or the eyelids, and/or the hands, and/or arms, and/or body, and/or elbows, of a person in need.

4. Cosmetic use according to any one of claims 1 to 3, wherein the biomass of interest is the *Atractylodes lancea* root dried, cut, triturated, and ground root.

5. Cosmetic use according to any one of claims 1 to 4, wherein the ALRE is obtained by an extraction process comprising the following steps:
a) contacting *Atractylodes lancea* root with the extraction solvent forming the extraction mixture,
b) extracting the *Atractylodes lancea* root with the extraction solvent at a temperature higher than room temperature, and optionally
c) macerating the extraction mixture at room temperature.

6. Cosmetic use according to any one of claims 1 to 5, wherein the ALRE is in the form of a solid extract.

7. Cosmetic use according to claim 6, wherein the solid extract comprises between 1000 and 15000 ppm, preferably between 2000 and 13000 ppm, more preferably between 3000 and 12000 ppm, and more preferably between 3500 and 11500 ppm of atractylodin, and between 5000 and 30000 ppm, preferably 6000 and 29000 ppm, and more preferably between 8000 and 28500 ppm of the total phenolic content.

8. Cosmetic use according to any one of claims 1 to 5, wherein the ALRE is in the form of a liquid extract.

9. Cosmetic use according to claim 8, wherein the liquid extract comprises between 0.5 wt% and 5 wt% of dry matter.

10. Cosmetic use according to claim 8 or 9, wherein the ALRE in the form of a liquid extract comprises between 50 and 300 ppm, preferably between 50 and 250 ppm, more preferably between 50 and 200 ppm, and yet more preferably between 50 and 150 ppm of atractylodin, and between 100 and 500 ppm, preferably between 100 and 475 ppm, and more preferably between 120 and 450 ppm of the total phenolic content.

11. Cosmetic use according to any one of claims 1 to 10, wherein the ALRE is present in a cosmetic composition.

12. Cosmetic use according to claim 11, wherein the cosmetic composition comprises the ALRE and a cosmetically acceptable component.

13. Cosmetic use according to claim 12, wherein the cosmetically acceptable component is selected from a cosmetically acceptable vehicle, a cosmetic ingredient, and mixtures thereof.

14. Cosmetic use according to any one of claims 11 to 13, wherein the cosmetic composition comprises:
a) between 0.001 wt% and 1.2 wt% of the ALRE, expressed as dry matter, preferably between 0.005 wt% and 0.85 wt% of the ALRE, more preferably between 0.008 wt% and 0.65 wt%, more preferably between 0.01 wt% and 0.12 wt%, and yet more preferably between 0.02 wt% and 0.06 wt%
b) between 5 wt% and 50 wt% of at least one additional cosmetically acceptable ingredient, is preferably between 10 wt% and 45 wt%, more preferably between 15 wt% and 35 wt%, and even more preferably between 20 wt% and 25 wt%, and
c) between 49 wt% and 94 wt% of a cosmetically acceptable vehicle, preferably between 50 wt% and 90 wt%, more preferably between 55 wt% and 87 wt%, and even more preferably between 65 wt% and 85 wt%,
where the percentages of the components are adjusted so that the balance is 100%.

15. Cosmetic method for skin anti-aging comprising the step of topically applying to skin the ALRE according to any one of claims 6 to 10, or a cosmetic composition according to any one of claims 11 to 14, wherein the ALRE is the only anti-aging active; preferably applying the ALRE or the cosmetic composition to the low neckline, and/or the face, and/or the cleavage, and/or the lips, and/or the cheeks, and/or the eyelids, and/or the hands.
